# EUROPÄISCHE PATENTANMELDUNG

(11) **EP 2 000 039 A1**
(43) Veröffentlichungstag der Anmeldung: **10.12.2008**
(21) Anmeldenummer: 08157690.2
(22) Anmeldetag: 05.06.2008
(51) Int. Cl.: A43B 13/12, A43B 13/18, A43B 13/38, A43B 17/00, A43B 17/02, B29D 31/515

(54) **Einlage für einen Schuh**

(30) Priorität: 05.06.2007 DE 202007008016 U
(71) Anmelder: Zimmermann, Manuela, 86343 Königsbrunn (DE); Riedner, Waltraud, 86343 Königsbrunn (DE)
(72) Erfinder: Zimmermann, Manuela, 86343 Königsbrunn (DE); Riedner, Waltraud, 86343 Königsbrunn (DE)
(74) Vertreter: Jannig, Peter

(57) **Zusammenfassung**

Es wird eine Einlage für einen Schuh beschrieben, wobei die Einlage
- einen Fersenbereich aufweist, auf welchem bei bestimmungsgemäßem Gebrauch der Einlage die Ferse des Fußes des Benutzers der Einlage zu liegen kommt,
- einen Längsgewölbebereich aufweist, auf welchem bei bestimmungsgemäßem Gebrauch der Einlage das Längsgewölbe des Fußes des Benutzers der Einlage zu liegen kommt, und
- einen Vorderfußbereich aufweist, auf welchem bei bestimmungsgemäßem Gebrauch der Einlage der Vorderfuß des Fußes des Benutzers der Einlage zu liegen kommt,

Die beschriebene Einlage zeichnet sich dadurch aus, daß sie im Fersenbereich und/oder im Längsgewölbebereich und/oder im Vorderfußbereich andere Eigenschaften aufweist als es bei dem den jeweils betreffenden Bereich umgebenden Bereich der Einlage der Fall ist.

## Beschreibung

Die vorliegende Erfindung betrifft eine Einlage für einen Schuh gemäß dem Oberbegriff des Patentanspruchs 1.

Einlagen für Schuhe sind plattenförmige Gebilde, die sich in Schuhe einlegen lassen und im in einen Schuh eingelegten Zustand auf der Sohle des Schuhs, genauer gesagt zwischen der Sohle des Schuhs und dem Fuß der die Einlage für den Schuh verwendenden Person zu liegen kommen. Einlagen für Schuhe, im Folgenden der Einfachheit halber nur kurz als Einlage bezeichnet, können für die unterschiedlichsten Zwecke verwendet werden. Eine erste Einsatzmöglichkeit von Einlagen besteht darin, daß der Schuh zu groß ist und durch die Einlage so weit verkleinert wird, daß er auch von Personen getragen werden kann, für die der Schuh ohne die Verwendung einer Einlage zu groß wäre. Eine weitere Einsatzmöglichkeit von Einlagen besteht in der Erhöhung des Tragekomforts von Schuhen. Die Sohle des Schuhs weist nämlich häufig Eigenschaften auf, die nicht oder jedenfalls nur in den seltensten Fällen exakt den individuellen Vorlieben der den Schuh benutzenden Person entsprechen. Die Sohle kann beispielsweise zu weich oder zu hart sein, kein oder ein schlecht an die Fußform angepaßtes Fußbett aufweisen, oder mit einem unangenehm zu tragenden Material bezogen sein. Die Verwendung von Einlagen bietet dem Benutzer des Schuhs die Möglichkeit, unter der riesigen Anzahl von verfügbaren Einlagen weitestgehend unabhängig vom Schuh eine einen höheren Tragekomfort bietende Einlage auszusuchen. Eine weitere Einsatzmöglichkeit von Einlagen besteht darin, daß durch Einlagen die unangenehmen Auswirkungen von Erkrankungen oder Mißbildungen des Fußes (Plattfuß, Senkfuß, Spreizfuß, Klumpfuß etc.) gelindert werden können. Zwar lassen sich die genannten Erkrankungen oder Mißbildungen des Fußes durch Einlagen in der Regel nicht mehr rückgängig machen, doch ermöglichen sie bei Auswahl einer geeigneten Einlage ein schmerzfreies Gehen und können zudem eine weitere Verschlechterung des Gesundheitszustandes verhindern oder zumindest verzögern.

Trotz der riesigen Anzahl an verfügbaren Einlagen besteht noch ein weiterer Bedarf an Verbesserungen. Insbesondere wäre es wünschenswert, wenn sich der Tragekomfort noch weiter verbessern ließe.

Der vorliegenden Erfindung liegt daher die Aufgabe zugrunde, die Einlage gemäß dem Oberbegriff des Schutzanspruchs 1 derart weiterzubilden, daß diese einen höheren Tragekomfort bietet.

Diese Aufgabe wird erfindungsgemäß durch die im kennzeichnenden Teil des Patentanspruchs 1 beanspruchten Merkmale gelöst.

Die erfindungsgemäße Einlage zeichnet sich dadurch aus, daß sie im Fersenbereich und/oder im Längsgewölbebereich und/oder im Vorderfußbereich andere Eigenschaften aufweist als es bei dem den jeweils betreffenden Bereich umgebenden Bereich der Einlage der Fall ist.

Dadurch kann die erfindungsgemäße Einlage im Fersenbereich und/oder im Längsgewölbebereich und/oder im Vorderfußbereich, also an denjenigen Stellen, an welchen der Fuß des Benutzers beim Stehen, Gehen, und/oder Laufen besonders hohen Belastungen ausgesetzt ist, beispielsweise besondere Elastizitätseigenschaften aufweisen, und/oder besondere Dämpfungseigenschaften aufweisen und/oder besonders rutschfest sein. Dies schont den Fuß und erhöht den Tragekomfort.

Vorteilhafte Weiterbildungen der Erfindung sind der folgenden Beschreibung, den Figuren, und den Unteransprüchen entnehmbar.

Die Erfindung wird nachfolgend anhand von Ausführungsbeispielen unter Bezugnahme auf die Figuren näher erläutert. Es zeigen
- Figur 1: eine Seitenansicht eines ersten Ausführungsbeispiels der erfindungsgemäßen Einlage,
- Figur 2: eine Draufsicht auf das in der Figur 1 gezeigte Ausführungsbeispiel der erfindungsgemäßen Einlage von oben,
- Figur 3: eine Schnittansicht des in den Figuren 1 und 2 gezeigten Ausführungsbeispiels der erfindungsgemäßen Einlage längs der in der Figur 2 gezeigten Schnittlinie III-III,
- Figur 4: eine Schnittansicht des in den Figuren 1 bis 3 gezeigten Ausführungsbeispiels der erfindungsgemäßen Einlage längs der in der Figur 2 gezeigten Schnittlinie IV-IV,
- Figur 5: eine Schnittansicht des in den Figuren 1 bis 4 gezeigten Ausführungsbeispiels der erfindungsgemäßen Einlage längs der in der Figur 2 gezeigten Schnittlinie V-V,
- Figur 6: eine Schnittansicht des in den Figuren 1 bis 5 gezeigten Ausführungsbeispiels der erfindungsgemäßen Einlage längs der in der Figur 2 gezeigten Schnittlinie VI-VI,
- Figur 7: eine Draufsicht auf ein zweites Ausführungsbeispiel der erfindungsgemäßen Einlage von oben,
- Figur 8: eine Seitenansicht der obersten Schicht des in der Figur 7 gezeigten zweiten Ausführungsbeispiels der erfindungsgemäßen Einlage, und

- Figur 9: eine Draufsicht auf eine nach einem besonderen Verfahren hergestellten Einlage gemäß den Figuren 1 bis 6.

Die erfindungsgemäße Einlage enthält
- einen Fersenbereich, auf welchem bei bestimmungsgemäßem Gebrauch der Einlage die Ferse des Fußes des Benutzers der Einlage zu liegen kommt,
- einen Längsgewölbebereich, auf welchem bei bestimmungsgemäßem Gebrauch der Einlage das Längsgewölbe des Fußes des Benutzers der Einlage zu liegen kommt, und
- einen Vorderfußbereich, auf welchem bei bestimmungsgemäßem Gebrauch der Einlage der Vorderfuß des Fußes des Benutzers der Einlage zu liegen kommt,
   und weist im Fersenbereich und/oder im Längsgewölbebereich und/oder im Vorderfußbereich andere Eigenschaften auf als es bei dem den jeweils betreffenden Bereich umgebenden Bereich der Einlage der Fall ist.

Im Folgenden werden mehrere Ausführungsbeispiele einer derartigen Einlage beschrieben.

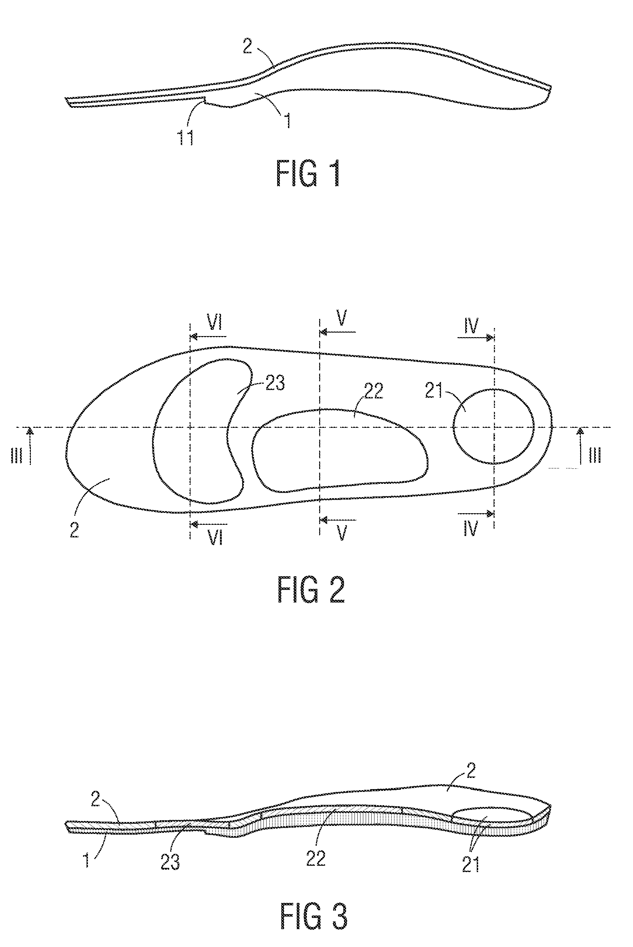

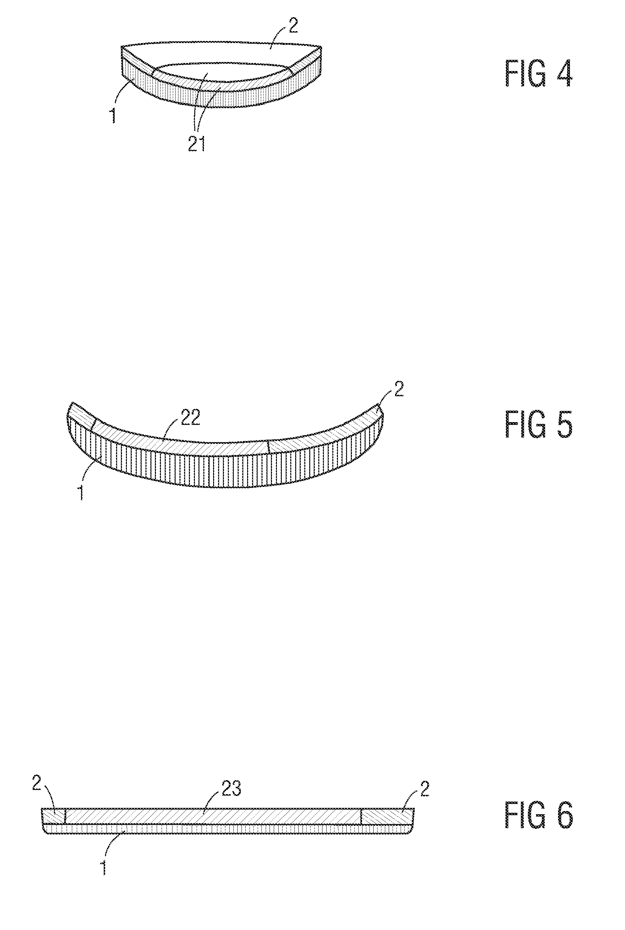

Das erste Ausführungsbeispiel ist in den Figuren 1 bis 6 veranschaulicht.

Wie insbesondere aus der Figur 1 ersichtlich ist, besteht das erste Ausführungsbeispiel der Einlage aus zwei übereinander angeordneten und beispielsweise durch Kleben miteinander verbundenen Schichten, genauer gesagt einer unteren Schicht 1 und einer darüber angeordneten oberen Schicht 2. Es sei bereits an dieser Stelle erwähnt, daß sie Erfindung auch bei Einlagen zu Einsatz kommen kann, die aus mehr oder weniger als zwei Schichten bestehen.

Die untere Schicht 1 ist im betrachteten Beispiel nochmals aus mehreren Schichten aufgebaut, die jedoch in den Figuren nicht gezeigt sind. Sie besteht im betrachteten Beispiel aus einem steifen Kern, und einer den Kern umgebenden, aus elastischerem Material bestehenden Ummantelung. Der Kern ist so steif, daß er sich nicht oder nur mit großem Kraftaufwand verformen läßt. Er besteht im betrachteten Beispiel aus einer Kunststoffplatte, könnte aber auch aus Metall oder einem sonstigen Material bestehen, das eine entsprechend hohe Festigkeit aufweist. Der Kern bestimmt die Form der Einlage, insbesondere die Form des auf der Oberseite der Einlage vorgesehenen Fußbettes. Im betrachteten Beispiel weist die Einlage ein an die Form des Fußes angepaßtes Fußbett auf; die vorliegende Erfindung kann jedoch auch bei Einlagen zum Einsatz kommen, bei welchen dies nicht der Fall ist. Der Vollständigkeit halber sei angemerkt, daß sich der Kern nicht über die gesamte Länge der Einlage erstreckt, genauer gesagt sich nur vom hinteren Ende der Einlage bis ungefähr zum Vorderfußbereich der Einlage (bis kurz vor einer in der Figur 1 gezeigten Stufe 11 an der Unterseite der unteren Schicht 1 erstreckt. Die Ummantelung besteht im betrachteten Beispiel aus Weichschaum oder einem ähnliche Eigenschaften aufweisenden elastischem Material, kann grundsätzlich aber auch aus einem beliebigen anderen Material wie Stoff, Kork, etc. bestehen.

Die obere Schicht 2 besteht aus einem elastisch verformbaren Material wie beispielsweise Weichschaum. Sie ist im nicht mit der unteren Schicht 1 verbundenen Zustand so biegsam, daß sie ohne eine Anpassung an die Form der Oberseite der unteren Schicht 1 ohne Zwischenräume und Faltenbildung mit der unteren Schicht 1 verbunden werden kann, sich beim Aufbringen auf die untere Schicht 1 also vollständig an die Form der unteren Schicht 2 anpaßt. Im betrachteten Beispiel ist die obere Schicht 2 an allen Stellen gleich dick (im betrachteten Beispiel ca. 2 mm) und weist eine ebene Oberseite und eine ebene Unterseite auf.

Bei der fertigen Einlage sind in dieser auf deren Oberseite
- im Fersenbereich, auf welchem bei bestimmungsgemäßem Gebrauch der Einlage die Ferse des Fußes des Benutzers der Einlage zu liegen kommt, und/oder
- im Längsgewölbebereich, auf welchem bei bestimmungsgemäßem Gebrauch der Einlage das Längsgewölbe des Fußes des Benutzers der Einlage zu liegen kommt, und/oder
- im Vorderfußbereich, auf welchem bei bestimmungsgemäßem Gebrauch der Einlage der Vorderfuß des Fußes des Benutzers der Einlage zu liegen kommt,
   eingesetzte Einsätze vorhanden. Der im Fersenbereich eingesetzte Einsatz ist in den Figuren mit dem Bezugszeichen 21 bezeichnet, der im Längsgewölbebereich eingesetzte Einsatz ist in den Figuren mit dem Bezugszeichen 22 bezeichnet, und der im Vorderfußbereich eingesetzte Einsatz ist in den Figuren mit dem Bezugszeichen 23 bezeichnet. Die Einsätze 21, 22, 23 sind in entsprechende Aussparungen in der oberen Schicht 2 eingesetzt und schließen oben bündig mit der Oberseite der oberen Schicht 2 ab.

Die in der oberen Schicht 2 für die Einsätze 21, 22, 23 vorgesehenen Aussparungen erstrecken sich im betrachteten Beispiel durch die gesamte obere Schicht 2, sind also in der oberen Schicht 2 vorhandene Löcher. Diese Löcher können beispielsweise durch Stanzen oder durch entsprechende Aussparungen bei der Herstellung der oberen Schicht 2 erzeugt werden.

Obgleich es in der Praxis am einfachsten sein dürfte, daß sich die für die Einsätze 21, 22, 23 vorgesehenen Aussparungen durch die gesamte obere Schicht 2 erstrecken, besteht hierfür kein zwingende Notwendigkeit. Es könnte auch vorgesehen werden, daß die Aussparungen die obere Schicht 2 nur teilweise durchdringen. Derartige Aussparungen können beispielsweise durch Fräsen oder durch Vorsehen entsprechender Vertiefungen bei der Herstellung der oberen Schicht 2 erzeugt werden.

Ebenso könnte vorgesehen werden, daß sich die Einsätze bis in die untere Schicht 1 oder in zwischen der unteren Schicht 1 und der oberen Schicht 2 gegebenenfalls vorhandene weitere Schichten erstrecken.

Die in die Aussparungen eingesetzten Einsätze 21, 22, 23 weisen andere Eigenschaften auf als die obere Schicht 2. Sie können beispielsweise, aber nicht ausschließlich ein anderes Elastizitätsverhalten, und/oder andere Dämpfungseigenschaften, und/oder unterschiedliche Reibungseigenschaften aufweisen.

Die Einsätze können beispielsweise aus Silikon bestehen. Zur Herstellung und Befestigung solcher Einsätze existieren unterschiedliche Möglichkeiten. Eine Möglichkeit besteht darin, daß in die Aussparungen flüssiges Silikon gefüllt wird, welches dann unter Rütteln und Aufdrücken eines entsprechend geformten Stempels durch einen Vulkanisationsprozeß aushärtet und sich mit der Einlage verbindet. Eine weitere Möglichkeit besteht darin, daß entsprechend geformte Silikon-Einsätze in die Aussparungen eingeklebt werden. Ferner könnte vorgesehen werden, die Aussparung so auszubilden, daß sie an dem der unteren Schicht 1 zugewandten Ende größer ist als an dem von der unteren Schicht 1 abgewandten Ende und einen entsprechend geformten Silikon-Einsatz vor dem Verbinden der oberen Schicht 1 mit der unteren Schicht 2 von unten in die obere Schicht 1 einzusetzen.

Die Einsätze können auch aus einem von der Firma Technogel GmbH & Co. KG, Max-Näder-Straße 15, D-37115 Duderstadt unter der Bezeichnung "Technogel" erhältlichen Material bestehen. Dies ist ein besonders flexibles und hochelastisches Material auf Polyurethan-Basis.

Die Einsätze können auch aus beliebigen anderen Materialien bestehen. Welches Material am besten geeignet ist, hängt auch davon ab, welche besonderen Eigenschaften die Einlage im Fersenbereich und/oder im Längsgewölbebereich und/oder im Vorderfußbereich aufweisen soll. Im allgemeinen erweist es sich als vorteilhaft, wenn die Einlage an den genannten Stellen besonders gute Dämpfungseigenschaften aufweist, weil dies die Füße des Einlagenbenutzers schont und einen hohen Tragekomfort bietet. Als vorteilhaft erweist es sich ferner, wenn die Einsätze einen hohen Reibungswiderstand aufweisen, so daß die Füße des Benutzers sich nicht oder nur in geringem Umfang relativ zur Einlage bewegen können. Dadurch läßt sich die Gefahr der Entstehung von Blasen an den Füßen verringern. Auch die Verwendung von Einsätzen, die mit geringerem Kraftaufwand und/oder in größerem Umgang elastisch verformbar sind als es bei dem den jeweils betreffenden Bereich umgebenden Bereich der Einlage der Fall ist, kann sich als vorteilhaft erweisen. Dies ermöglicht es, daß Personen mit einem Fersensporn schmerzfrei gehen oder laufen können. Diese Aufzählung ließe sich beliebig fortsetzen und zeigt, welche neuen Möglichkeiten es bietet, wenn man Einlagen verwendet, die im Fersenbereich und/oder im Längsgewölbebereich und/oder im Vorderfußbereich andere Eigenschaften aufweisen als es bei dem den jeweils betreffenden Bereich umgebenden Bereich der Einlage der Fall ist. Der Umstand, daß die Einlage lokal unterschiedliche Eigenschaften aufweist, vereinfacht es, die Einlage an die individuellen Bedürfnisse des Benutzers anzupassen. Dies kann auch die Benutzung der Einlage erleichtern. Beispielsweise kann es sich als schwierig erweisen, mit dem Fuß in einen Schuh zu kommen, von dessen Einlage die gesamte obere Schicht aus Silikon besteht. Silikon hat nämlich einen sehr großen Reibungswiderstand, so daß ein schnelles Einsteigen in einen Schuh mit einer solchen Einlage mit großen Schwierigkeiten verbunden sein kann. Die nur lokale Ausbildung der oberen Einlagenschicht mit Silikon vermag diese Probleme erheblich zu reduzieren. Ferner läßt sich die erfindungsgemäße Einlage unter Umständen auch einfacher und/oder billiger herstellen.

Die Eigenschaften der Einlage, insbesondere die besonderen Eigenschaften der Einlage im Fersenbereich und/oder im Längsgewölbebereich und/oder im Vorderfußbereich lassen sich durch das für die Einsätze 21, 22, 23 verwendete Material und die gewählte Dicke der Einsätze 21, 22, 23 (und der Tiefe der zugeordneten Aussparungen in der oberen Schicht 2) auf einfache Weise wunschgemäß einstellen.Dabei kann es sich auch als vorteilhaft erweisen, wenn die Einsätze 21, 22, 23 zumindest teilweise unterschiedliche Eigenschaften aufweisen, also beispielsweise unterschiedlich dick ausgebildet sind und/oder aus unterschiedlichen Materialien hergestellt sind. Dadurch können die Eigenschaften der Einlage im Fersenbereich, im Längsgewölbebereich, und im Vorderfußbereich unabhängig voneinander festgelegt werden. Darüber hinaus besteht auch keine zwingende Notwendigkeit, sowohl im Fersenbereich als auch im Längsgewölbebereich als auch im Vorderfußbereich Einsätze 21, 22, 23 vorzusehen. Auch Einlagen, bei welchen nur in einem oder zwei der genannten Einlagenbereiche Einsätze vorgesehen sind, sind Einlagen ohne die beschriebenen Einsätze überlegen. Für bestimmte Anwendungsfälle kann es sogar von Vorteil sein, wenn nicht sowohl im Fersenbereich als auch im Längsgewölbebereich als auch im Vorderfußbereich Einsätze vorgesehen sind. Unabhängig hiervon kann es sich auch als vorteilhaft erweisen, wenn nicht nur im Fersenbereich und/oder im Längsgewölbebereich und/oder im Vorderfußbereich, sondern zusätzlich oder alternativ auch in anderen Einlagenbereichen Einsätze der beschriebenen Art vorgesehen sind. Vorstehendes gilt nicht nur für das vorstehend beschriebene erste Ausführungsbeispiel der hier vorgestellten Einlage, sondern auch für die im Folgenden beschriebenen weiteren Einlagen-Ausführungsbeispiele.

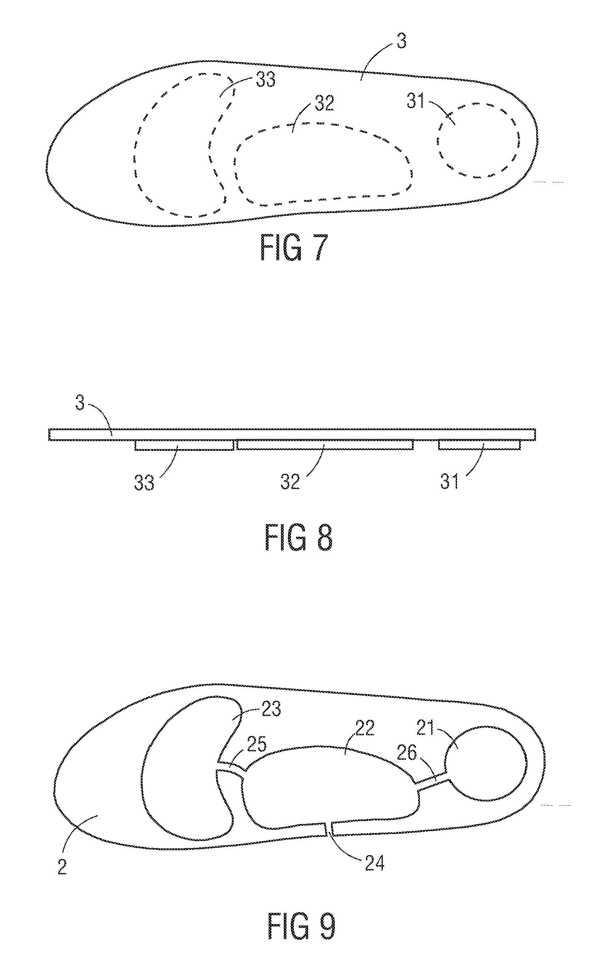

Die erfindungsgemäße Einlage kann auch wie im Folgenden unter Bezugnahme auf die Figuren 7 und 8 beschrieben aufgebaut sein.

Das in den Figuren 7 und 8 gezeigte zweite Ausführungsbeispiel der erfindungsgemäßen Einlage weist wie das vorstehend unter Bezugnahme auf die Figuren 1 bis 6 beschriebene erste Ausführungsbeispiel eine untere Schicht und eine darüber angeordnete obere Schicht auf, wobei die untere Schicht der unteren Schicht 1 des ersten Einlagen-Ausführungsbeispiels entspricht und die obere Schicht der oberen Schicht 2 des ersten Einlagen-Ausführungsbeispiels entspricht. Im Fersenbereich und/oder im Längsgewölbebereich und/oder im Vorderfußbereich der oberen Schicht 2 sind auch wiederum Aussparungen für Einsätze vorgesehen. Das zweite Ausführungsbeispiel der erfindungsgemäßen Einlage entspricht insoweit dem ersten Ausführungsbeispiel. Zur Vermeidung von Wiederholungen wird bezüglich der weiteren Einzelheiten zur unteren Schicht, zur oberen Schicht und zu den in der oberen Schicht vorgesehenen Aussparungen auf die entsprechenden Ausführungen in der Beschreibung des ersten Ausführungsbeispiels verwiesen. Die in der oberen Schicht vorgesehenen Aussparungen sind auch wiederum zum Einsetzen von Einsätzen vorgesehen. Allerdings weisen diese Einsätze einen anderen Aufbau auf als es beim ersten Einlagen-Ausführungsbeispiel der Fall ist.

Beim zweiten Ausführungsbeispiel sind die Einsätze Bestandteil einer über der oberen Schicht angeordneten, in den Figuren 7 (Draufsicht von oben) und 8 (Seitenansicht) gezeigten dritten Schicht 3. Die Einsätze sind als nach unten ragende Erhebungen 31, 32, 33 der dritten Schicht 3 ausgebildet. Die Erhebungen 31, 32, 33 gehen im betrachteten Beispiel rechtwinklig von der Unterseite der dritten Schicht 3 ab. Die seitlichen Flanken können aber auch einen beliebigen anderen Winkel zur Unterseite der dritten Schicht 3 aufweisen. Die dritte Schicht 3 wird über der oberen Schicht 2 angeordnet und bedeckt diese vollständig. Dabei kommen die Erhebungen 31, 32, 33 in den dafür vorgesehenen Aussparungen in der oberen Schicht 2 zu liegen. Genauer gesagt kommt die Erhebung 31 in der im Fersenbereich der Einlage vorgesehenen Aussparung zu liegen, die Erhebung 32 in der im Längsgewölbebereich der Einlage vorgesehenen Aussparung zu liegen, und die Erhebung 33 in der im Vorderfußbereich der Einlage vorgesehenen Aussparung zu liegen.

Die dritte Schicht 3 besteht aus einem elastisch verformbaren Material wie beispielsweise dem vorstehend bereits erwähnten Material Technogel. Sie ist im nicht mit der oberen Schicht 2 verbundenen Zustand so biegsam, daß sie ohne eine Anpassung an die Form der Oberseite der oberen Schicht 2 ohne Zwischenräume und Faltenbildung mit der oberen Schicht 2 verbunden werden kann, sich beim Aufbringen auf die obere Schicht 2 also vollständig an die Form der oberen Schicht 2 anpaßt. Im betrachteten Beispiel ist die dritte Schicht 3 in den Bereichen, in welchen keine Erhebungen vorgesehen sind, überall gleich dick (im betrachteten Beispiel ca. 1 bis 2 mm) und weist eine ebene Oberseite auf. Auch in den Bereichen, in welchen die Erhebungen 31, 32, 33 vorgesehen sind, weist die dritte Schicht eine konstante Dicke auf, wobei diese Dicke jedoch um die Höhe der Erhebungen 31, 32, 33 größer ist als die Dicke der dritten Schicht 3 in den erhebungsfreien Bereichen.

Die dritte Schicht 3 kann auch aus einem beliebigen anderen Material bestehen.

Da die dritte Schicht 3 aufgrund der Erhebungen 31, 32, 33 im Fersenbereich und/oder im Längsgewölbebereich und/oder im Vorderfußbereich dicker ausgebildet ist als in den restlichen Bereichen ist es auch beim zweiten Einlagen-Ausführungsbeispiel so, daß die Einlage im Fersenbereich und/oder im Längsgewölbebereich und/oder im Vorderfußbereich andere Eigenschaften aufweist als es bei dem den jeweils betreffenden Bereich umgebenden Bereich der Einlage der Fall ist.

Durch das zweite Einlagen-Ausführungsbeispiel lassen sich die selben Vorteile erzielen wie durch das erste Einlagen-Ausführungsbeispiel.

Die Eigenschaften der Einlage, insbesondere die besonderen Eigenschaften der Einlage im Fersenbereich und/oder im Längsgewölbebereich und/oder im Vorderfußbereich lassen sich durch das für die dritte Schicht 3 verwendete Material und die gewählte Höhe der Erhebungen 31, 32, 33 (und der Tiefe der zugeordneten Aussparungen in der oberen Schicht 2) auf einfache Weise wunschgemäß einstellen.

Ein drittes Einlagen-Ausführungsbeispiel besteht darin, daß die im zweiten Einlagen-Ausführungsbeispiel verwendete dritte Schicht 3 alleine als Einlage verwendet wird und mit den Erhebungen 31, 32, 33 nach unten in einen Schuh eingelegt wird. Um einer solchen Einlage eine höhere Stabilität zu verleihen, kann bei Bedarf vorgesehen werden, die erhebungsfreien Bereiche der Schicht 3 dicker auszubilden als es der Fall ist, wenn die Schicht 3 wie beim zweiten Einlagen-Ausführungsbeispiel verwendet wird.

Da das dritte Einlagen-Ausführungsbeispiel aufgrund der Erhebungen 31, 32, 33 im Fersenbereich und/oder im Längsgewölbebereich und/oder im Vorderfußbereich dicker ausgebildet ist als in den restlichen Bereichen ist es auch beim dritten Einlagen-Ausführungsbeispiel so, daß die Einlage im Fersenbereich und/oder im Längsgewölbebereich und/oder im Vorderfußbereich andere Eigenschaften aufweist als es bei dem den jeweils betreffenden Bereich umgebenden Bereich der Einlage der Fall ist.

Durch das dritte Einlagen-Ausführungsbeispiel lassen sich die selben Vorteile erzielen wie durch das erste Einlagen-Ausführungsbeispiel und das zweite Einlagen-Ausführungsbeispiel.

Die Eigenschaften der Einlage, insbesondere die besonderen Eigenschaften der Einlage im Fersenbereich und/oder im Längsgewölbebereich und/oder im Vorderfußbereich lassen sich durch das für Einlage verwendete Material und die gewählte Höhe der Erhebungen 31, 32, 33 auf einfache Weise wunschgemäß einstellen.

Abschließend wird unter Bezugnahme auf die Figur 9 noch eine besonders elegante Möglichkeit zur Herstellung der Einlage gemäß dem eingangs beschriebenen ersten Ausführungsbeispiel beschrieben.

Bei diesem Herstellungsverfahren wird in einem ersten Schritt die obere Schicht 2 der Einlage mit den für die Einsätze 21, 22, 23 vorzusehenden Vertiefungen, aber noch ohne die Einsätze 21, 22, 23 im Spritzgießverfahren hergestellt. Dabei wird eine Spritzgießform verwendet wird, bei welcher sich
- an den Stellen, an welchen sich bei der fertigen Einlage die Einsätze 21, 22, 23 befinden, und
- an den in der Figur 9 mit den Bezugszeichen 24, 25, 26 bezeichneten Stellen

Erhebungen befinden, durch welche bewirkt wird, daß in dem durch das Spritzgießen hergestellten Teil der oberen Schicht 2 entsprechende Vertiefungen ausgebildet sind.

Die Vertiefungen an den in der Figur 9 mit den Bezugszeichen 24, 25, 26 bezeichneten Stellen bilden während eines später noch genauer beschriebenen weiteres Arbeitsschrittes Kanäle, deren Funktion später noch genauer beschrieben wird.

Die Vertiefungen erstrecken sich im betrachteten Beispiel nicht über die gesamte Dicke der oberen Schicht 2, sind also keine durchgehenden Löcher. Obgleich dies die derzeit bevorzugte Variante ist, kann auch vorgesehen werden, daß sich die Vertiefungen durch die gesamte obere Schicht 2 hindurch erstrecken, also durch durchgehende Löcher gebildet werden.

Anschließend wird, ohne den im ersten Herstellungsschritt erzeugten Teil der oberen Schicht 2 aus der Spritzgießform zu entnehmen, der die genannten Erhebungen aufweisende Teil der Spritzgießform durch eine Platte ohne die genannten Erhebungen ersetzt, wodurch an den Stellen, an welchen sich in der ersten Spritzgießform die Erhebungen befanden, Hohlräume entstehen.

Diese Hohlräume werden in einem weiteren Schritt mit dem später die Einsätze bildenden, anderen Kunststoff ausgefüllt. Genauer gesagt wird in den Kanal 24 flüssiger Kunststoff eingefüllt, welcher über den Kanal 24 in die für den Einsatz 22 vorgesehene Vertiefung und von dort weiter über den Kanal 25 in die für den Einsatz 23 vorgesehene Vertiefung und über den Kanal 26 in die für den Einsatz 21 vorgesehene Vertiefung gelangt. Das Einfüllen von Kunststoff in den Kanal 24 wird so lange fortgesetzt, bis die vorstehend erwähnten Hohlräume vollständig mit diesem Kuststoff ausgefüllt sind.

Beim Aushärten des im zweiten Schritt eingefüllten Kunststoffes geht dieser eine feste Verbindung mit dem im ersten Schritt hergestellten Teil der oberen Schicht 2 ein. Der die Vertiefungen für die Einsätze 21, 22, 23 ausfüllende Kunststoff bildet die Einsätze 21, 22, 23.

Damait ist die Herstellung der oberen Schicht 2 der Einlage abgeschlossen. Die obere Schicht 2 kann anschließend auf herkömmliche Art und Weise mit der unteren Schicht 1 verbunden werden.

Der Vollständigkeit halber sei angemerkt, daß der Kanal 24 anstatt in die Vertiefung für den Einsatz 22 auch in die Vertiefung für den Einsatz 21 oder in die Vertiefung für den Einsatz 23 münden könnte.

Es könnte auch vorgesehen werden, daß der Kanal 24 sowohl in die Vertiefung für den Einsatz 21 als auch in die Vertiefung für den Einsatz 22 als auch in die Vertiefung für den Einsatz 23 mündet. In diesem fall könnte auf die Kanäle 25 und 26 verzichtet werden.

Ferner könnte vorgesehen werden, die Vertiefung für den Einsatz 21 und/oder die Vertiefung für den Einsatz 22 und/oder die Vertiefung für den Einsatz 23 von oben oder unten mit Kunststoff zu befüllen, wodurch zumindest auf den Kanal 24 und je nach Ausführungsform gegebenenfalls auch auf den Kanal 25 und/oder den Kanal 26 verzichtet werden kann.

Bei einer wie beschrieben hergestellten Einlage ergibt sich eine besonders feste und dauerhafte Verbindung zwischen den Einsätzen und dem Rest der oberen Schicht 2. Darüber hinaus sind zur Herstellung der Einlage nur wenige Arbeitsschritte erforderlich, die sich zudem mit sehr geringem Aufwand und höchster Präzision vollautomatisch durchführen lassen.

Noch einfacher gestaltet sich die Herstellung einer Einlage der in der Figuren 1 bis 6 gezeigten Art, wenn im ersten Arbeitsschritt des vorstehend beschriebenen Herstellungsverfahrens die vorgefertigte untere Schicht 1 der Einlage gleich in die (entsprechend modifizierte) Spritzgießform eingelegt wird. Dann wird bei diesem ersten Arbeitsschritt nicht nur die obere Schicht 2 hergestellt, sondern gleichzeitig auch die obere Schicht 2 mit der unteren Schicht 1 verbunden.

Diese vereinfachte (erste) Herstellungsalternative kann noch weiter vereinfacht werden, indem zwar die selbe Spritzgießform verwendet wird wie es bei der ersten Herstellungsalternative erforderlich wäre, aber keine untere Schicht 1 in die Spritzgießform eingelegt wird. Dadurch erhält man durch den ersten Arbeitsschritt ein Zwischenprodukt, das die selbe Form hat wie das unter Verwendung der ersten Herstellungsalternative hergestellte Zwischenprodukt, aber nur aus einer einzigen Schicht besteht und keinen steifen Kern enthält. Das Fehlen des steifen Kerns kann bei Bedarf dadurch ausgeglichen werden, daß der im ersten Arbeitsschritt verwendete Kunststoff ein mehr oder weniger härterer Kunststoff ist.

Die nach der zweiten Herstellungsalternative hergestellten Einlagen haben die selben vorteilhaften Eigenschaften wie die nach einem der anderen beschriebenen Herstellungsverfahren hergestellten Einlagen, lassen sich aber noch einfacher, schneller, und kostengünstiger herstellen als es bei den anderen beschriebenen Herstellungsverfahren der Fall ist.

Die vorstehend beschriebenen Einlagen erweisen sich unabhängig von der praktischen Realisierung derselben als vorteilhaft. Diese Art von Einlagen ermöglicht es auf einfache Weise, den Einlagen im Fersenbereich und/oder im Längsgewölbebereich und/oder im Vorderfußbereich, also an denjenigen Stellen, an welchen der Fuß des Benutzers beim Stehen, Gehen, und/oder Laufen besonders hohen Belastungen ausgesetzt ist, besondere Eigenschaften zu verleihen.

### Bezugszeichenliste

- 1: untere Schicht
- 3: obere Schicht
- 3: dritte Schicht

- 11: Stufe

- 21: Einsatz im Fersenbereich
- 22: Einsatz im Längsgewölbebereich
- 23: Einsatz im Vorderfußbereich
- 24: Kanal
- 25: Kanal
- 26: Kanal

- 31: Erhebung im Fersenbereich
- 32: Erhebung im Längsgewölbebereich
- 33: Erhebung im Vorderfußbereich

## Patentansprüche

1. Einlage für einen Schuh, wobei die Einlage
- einen Fersenbereich aufweist, auf welchem bei bestimmungsgemäßem Gebrauch der Einlage die Ferse des Fußes des Benutzers der Einlage zu liegen kommt,
- einen Längsgewölbebereich aufweist, auf welchem bei bestimmungsgemäßem Gebrauch der Einlage das Längsgewölbe des Fußes des Benutzers der Einlage zu liegen kommt, und
- einen Vorderfußbereich aufweist, auf welchem bei bestimmungsgemäßem Gebrauch der Einlage der Vorderfuß des Fußes des Benutzers der Einlage zu liegen kommt,
**dadurch gekennzeichnet,**
**daß** die Einlage im Fersenbereich und/oder im Längsgewölbebereich und/oder im Vorderfußbereich andere Eigenschaften aufweist als es bei dem den jeweils betreffenden Bereich umgebenden Bereich der Einlage der Fall ist.

2. Einlage nach Anspruch 1,
**dadurch gekennzeichnet ,**
**daß** die Einlage im Fersenbereich und/oder im Längsgewölbebereich und/oder im Vorderfußbereich
- andere Elastizitätseigenschaften aufweist, und/oder
- mit geringerem Kraftaufwand elastisch verformbar ist, und/oder
- in größerem Umgang elastisch verformbar ist, und/oder
- andere Dämpfungseigenschaften aufweist, und/oder
- einen anderen Reibungswiderstand aufweist
als es bei dem den jeweils betreffenden Bereich umgebenden Bereich der Einlage der Fall ist.

3. Einlage nach Anspruch 1,
**dadurch gekennzeichnet ,**
**daß** die Einlage einen Grundkörper (1, 2) und in entsprechende Aussparungen im Grundkörper (1, 2) eingesetzte Einsätze (21, 22, 23, 31, 32, 33) umfaßt, wobei zumindest im Fersenbereich und/oder im Längsgewölbebereich und/oder im Vorderfußbereich Einsätze (21, 22, 23, 31, 32, 33) vorhanden sind, und wobei die Einsätze (21, 22, 23, 31, 32, 33) andere Eigenschaften aufweisen und/oder aus einem anderen Material bestehen als die die Einsätze umgebenden Bereiche der Einlage.

4. Einlage nach Anspruch 3,
**dadurch gekennzeichnet ,**
**daß** der Grundkörper aus mehreren übereinander angeordneten Schichten (1, 2) besteht, und daß die Aussparungen, in welche die Einsätze (21, 22, 23, 31, 32, 33) eingesetzt sind, in der obersten Schicht (2) des Grundkörpers (1, 2) vorgesehene Vertiefungen oder Löcher sind.

5. Einlage nach Anspruch 3 oder 4,
**dadurch gekennzeichnet ,**
**daß** die Einsätze (21, 22, 23, 31, 32, 33) aus einem flexiblen elastischen Material bestehen.

6. Einlage nach nach einem der Ansprüche 3 bis 5,
**dadurch gekennzeichnet ,**
**daß** die Einsätze (21, 22, 23, 31, 32, 33) aus Kunststoff wie insbesondere Silikon oder Technogel bestehen.

7. Einlage nach nach einem der Ansprüche 3 bis 6,
**dadurch gekennzeichnet ,**
**daß** die Einsätze (21, 22, 23, 31, 32, 33) oben bündig mit der Schicht (2) abschließen, in welche sie eingesetzt sind.

8. Einlage nach Anspruch 1,
**dadurch gekennzeichnet ,**
**daß** die Einlage aus mehreren übereinander angeordneten Schichten (1, 2, 3) besteht, wobei eine der Schichten (3) im Fersenbereich und/oder im Längsgewölbebereich und/oder im Vorderfußbereich der Einlage dicker ausgebildet ist als in den restlichen Bereichen der Einlage.

9. Einlage nach Anspruch 8,
**dadurch gekennzeichnet ,**
- **daß** die im Fersenbereich und/oder im Längsgewölbebereich und/oder im Vorderfußbereich der Einlage dicker ausgebildete Schicht (3) die oberste Schicht der Einlage ist,
- **daß** die Verdickungen der im Fersenbereich und/oder im Längsgewölbebereich und/oder im Vorderfußbereich der Einlage dicker ausgebildeten Schicht (3) durch von der Unterseite der betreffenden Schicht (3) nach unten abgehende Erhebungen (31, 32, 33) gebildet werden, und
- **daß** die nach unten abgehenden Erhebungen (31, 32, 33) der im Fersenbereich und/oder im Längsgewölbebereich und/oder im Vorderfußbereich der Einlage dicker ausgebildeten Schicht (3) in entsprechenden Aussparungen der darunter angeordneten Schicht (2) zu liegen kommen.

10. Einlage nach Anspruch 1,
**dadurch gekennzeichnet ,**
**daß** die Einlage ein plattenförmiges Gebilde ist, das im Fersenbereich und/oder im Längsgewölbebereich und/oder im Vorderfußbereich dicker ausgebildet ist als in der restlichen Bereichen.

11. Einlage nach Anspruch 1,
**dadurch gekennzeichnet ,**
**daß** die Oberseite der Einlage ein an die Form des Fußes des Einlagenbenutzers angepaßtes Fußbett aufweist.

12. Einlage nach Anspruch 11,
**dadurch gekennzeichnet ,**
**daß** der Fersenbereich und/oder der Längsgewölbebereich und/oder der Vorderfußbereich der Einlage als unebene Bereiche ausgebildet sind.

13. Einlage nach Anspruch 4 oder 8,
**dadurch gekennzeichnet ,**
**daß** eine der Schichten (1, 2) einen steifen Kern enthält.

14. Verfahren zur Herstellung einer Einlage gemäß den Ansprüchen 3 bis 7,
**dadurch gekennzeichnet ,**
- **daß** in einem ersten Arbeitsschritt unter Verwendung eines Spritzgießverfahrens der die Aussparungen für die Einsätze enthaltende Teil der Einlage hergestellt wird, und
- **daß** in einem weiteren Arbeitsschritt unter Verwendung eines Spritzgießverfahrens, bei welchem die Aussparungen mit einem anderen Kunststoff gefüllt werden, die Einsätze hergestellt werden.

15. Verfahren nach Anspruch 14,
**dadurch gekennzeichnet ,**
**daß** im ersten Arbeitsschritt außer den Aussparungen für die Einsätze weitere Aussparungen (24, 25, 26) gebildet werden, wobei diese weiteren Aussparungen im zweiten Arbeitsschritt Kanäle bilden, durch welche hindurch der später die Einsätze bildende Kuststoff in die Vertiefungen für die Einsätze gelangen kann.
